# EUROPÄISCHE PATENTSCHRIFT

(11) **EP 0 428 039 B1**
(45) Veröffentlichungstag und Bekanntmachung des Hinweises auf die Patenterteilung: **05.01.1994**
(21) Anmeldenummer: 90121191.2
(22) Anmeldetag: 06.11.1990
(51) Int. Cl.: C07C 19/08, C07C 17/20

(54) **Verfahren zur Herstellung von weitgehend fluorierten Alkylbromiden**
Process for the preparation of highly fluorinated alkylbromides
Procédé pour la préparation de bromures d'alkyle fortement fluorés

(30) Priorität: 11.11.1989 DE 3937567
(43) Veröffentlichungstag der Anmeldung: 22.05.1991
(73) Patentinhaber: HOECHST AKTIENGESELLSCHAFT, 65926 Frankfurt am Main (DE)
(72) Erfinder: Raab, Klaus, Dr., W-8269 Burgkirchen (DE)

(56) Entgegenhaltungen:
- HOUBEN-WEYL: "Methoden der organische Chemie", Auflage 4, Band V/4: "Halogenverbindungen", 1960, Seiten 354-360, Georg Thieme Verlag, Stuttgart, DE
- CHEMICAL ABSTRACTS, Band 104, 17. März 1986, Seite 619, Zusammenfassung Nr. 88106p, Columbus, Ohio, US; & JP-A-60 184 033

## Beschreibung

Die Erfindung betrifft ein Verfahren zur Herstellung von weitgehend fluorierten Alkylbromiden aus Alkyliodiden.

Es ist aus US-PS 2,678,953 bekannt, Perfluoralkylbromide aus den trockenen Metallsalzen von Perfluoralkylcarbonsäuren durch Umsetzung mit Brom zu gewinnen, wobei die Anwendung von sichtbarem Licht den Umsatz steigert. Im einzigen Beispiel wird das bevorzugte Silbersalz verwendet. Dieses Verfahren ist aufwendig, da erst die Perfluoralkylcarbonsäure erzeugt werden muß, beispielsweise durch die bekannte Umsetzung von Perfluoralkyliodid mit SO₃ oder rauchender Schwefelsäure. Die Säure muß dann in das Metallsalz umgewandelt und dieses getrocknet werden. Ferner erfordert die Umsetzung mit dem giftigen und ätzenden Brom besondere Vorsicht und einen erhöhten apparativen Aufwand (Sicherheitsvorkehrungen, Korrosion). Außerdem geht bei der Reaktionskette eine CF₂-Gruppe des Perfluoralkyliodids verloren.

Es ist ferner aus US-PS 2,875,253 bekannt, einen mit Fluor, Brom und gegebenenfalls mit Chlor substituierten, niedrigen Kohlenwasserstoff als Telogen mit einem Fluor enthaltenden Olefin, das zusätzlich,Chloratome enthalten kann, in Gegenwart eines peroxidischen Polymerisations-Promotors zu telomerisieren. Als mögliche Telogene sind unter anderem CF₃Br, CF₂BrCl, CF₂Br₂, C₂F₅Br, C₂F₄BrCl, C₃F₆BrH und C₃F₆Br₂ genannt, unter einer Vielzahl möglicher Fluor enthaltender Olefine Tetrafluorethylen. Die Umsetzung dieser Verbindungen müßte zu weitgehend fluorierten Alkylbromiden im Sinne der vorliegenden Erfindung führen, jedoch ist kein Beispiel vorhanden, aus dem zu entnehmen wäre, unter welchen genauen Bedingungen und mit welchem Erfolg die Umsetzung mit Tetrafluorethylen durchführbar ist. In den Beispielen wird als Fluor enthaltendes Olefin immer CF₂=CFCl eingesetzt.

In einem Aufsatz von Long, Higgins, Mattrey, Mitten und Multer über Radiopake Fluorkohlenwasserstoffe (R.E. Banks, "Preparation, Properties and Industrial Applications of Organofluorine Compounds", 1982, Ellis Horwood Ltd. Publishers/Chichester, Seiten 139 bis 156) ist am Ende der Ausführungen (Seite 154 unten) erwähnt, daß das für die Untersuchungen verwendete Perfluor-n-hexylbromid bzw. Perfluor-iso-heptylbromid durch thermische Bromierung der entsprechenden Perfluoralkyliodide mit elementarem Brom erzeugt wurde, doch fehlen nähere Angaben. Zweifellos steigen die weiter oben bereits erwähnten apparativen Schwierigkeiten bei Anwendung von Brom, wenn thermisch, das heißt bei höheren Temperaturen, mit diesem aggressiven Stoff gearbeitet wird.

R. N. Haszeldine, J. Chem. Soc., 1953, Seiten 3 761 bis 3 768 sowie Huang Bingnan und Huang Weiyuan, Shanghai Inst. Org. Chem. Acad. Sinica, Huaxue Xuebao 42, Seiten 1 106 bis 1 108 (C.A. 102; 78312x), 1984 beschreiben die photochemische Bromierung von Perfluoralkyliodiden [Beispiele:R_{f}I oder Cl(CF₂)₄I] mit Brom unter UV-Strahlung. Nach 168 beziehungsweise 50 Stunden Umsetzung wird R_{f}Br oder Cl(CF₂)₄Br in sehr guter Ausbeute erhalten. Allerdings ist auch dieses Verfahren apparativ und energetisch aufwendig.

Schließlich ist aus JP-Kokai 60-184033-A2, 1985 (C.A. 104; 88106p) bekannt, CₙF₂ₙ₊₁Br (n = 6 bis 11) durch Umsetzung von CₙF₂ₙ₊₁I mit Brom in Gegenwart einer Radikale erzeugenden Verbindung (zum Beispiel Azodiisobutyronitril) zu erzeugen. Es wird so C₆F₁₃Br mit 40 % Ausbeute gewonnen. Die Anwendung des elementaren Broms erfordert auch hier apparative Vorkehrungen.

Aufgabe der vorliegenden Erfindung ist es, ein Verfahren bereitzustellen, das mit vergleichsweise wesentlich weniger giftigen, gefährlichen und korrosiven Stoffen ermöglicht, Perfluoralkylbromide zu erzeugen.

Das neue Verfahren zur Herstellung von weitgehend fluorierten Alkylbromiden aus Verbindungen der Formel

X-(CF₂)ₙ-I (I),

in der bedeuten: X = H, F, Cl, Br, I oder (CF₃)₂CF- und n = 1 bis 16, ist dadurch gekennzeichnet, daß in mindestens einem dipolaren, aprotischen Lösungsmittel 1 mol gebundenes Iodatom der Verbindung der Formel (I) mit 1 bis 4 mol Bromidionen, die als Salze mit mindestens einem der folgenden Kationen: Alkalimetall, Erdalkalimetall, Kupfer, substituiertes oder unsubstituiertes Ammonium vorliegen, bei 120 bis 200 °C unter normalem Atmosphärendruck oder unter dem autogenen Druck der Reaktionsmischung umgesetzt werden.

Verbindungen der obengenannten Formel (I) sind nach verschiedenen bekannten Verfahren herstellbar. Beispielsweise kann durch Umsetzung von Iod mit Iodpentafluorid und Tetrafluorethylen Perfluorethyliodid erzeugt werden, das seinerseits durch Telomerisation mit weiterem Tetrafluorethylen zu höheren Perfluoralkyliodiden umgesetzt werden kann. Analoge Verbindungen sind durch Einsatz entsprechender Ausgangsstoffe erhältlich. Teilweise sind Verbindungen der Formel (I) Marktprodukte.

Vorzugsweise werden solche Verbindungen der Formel (I) eingesetzt, in denen X = I ist und insbesondere solche, in denen X entweder (CF₃)₂CF- oder F bedeutet. Verbindungen der Formel (I), in denen n größer als 16 ist, ergeben im allgemeinen längere Reaktionszeiten und häufig schlechtere Ausbeuten, sie sind auch in der Regel weniger gut verwendbar. Wegen ihrer guten Anwendbarkeit sind Verbindungen der Formel (I) bevorzugt, in denen n 4 bis 12 und insbesondere 6 bis 8 bedeutet. Es können auch Mischungen von Verbindungen der Formel (I) eingesetzt werden, die unterschiedliche Substituenten X und/oder unterschiedliche Zahlen n aufweisen.

Erfindungsgemäß wird 1 mol gebundenes Iodatom der Verbindung der Formel (I) mit 1 bis 4 mol Bromidionen, die als Salze mit verschiedenen Kationen vorliegen können, umgesetzt. Werden je 1 mol Iodatom in der Verbindung der Formel (I) weniger als 1 mol Bromidion verwendet, werden schlechtere Ausbeuten erhalten. Prinzipiell können über 4 mol Bromidionen je 1 mol Iodatom in der Verbindung der Formel (I) eingesetzt werden, jedoch wird im allgemeinen hierdurch keine Ausbeuteverbesserung beobachtet, so daß dies ein unnötiger Aufwand ist. Vorzugsweise werden je 1 mol Iodatom in der Verbindung der Formel (I) 1,1 bis 2 mol Bromidionen verwendet.

Das Bromid kann als Salz des substituierten oder unsubstituierten Ammoniums vorliegen; als Substituenten am Stickstoff des Ammoniums kommen beispielsweise in Frage Alkylgruppen mit 1 bis 4 C-Atomen oder Hydroxyalkylgruppen mit 2 bis 4 C-Atomen. Ferner kann das Bromid als Salz mit Kupfer oder einem Erdalkalimetall, beispielsweise Calcium oder Magnesium sowie auch als Salz mit einem Alkalimetall vorliegen. Wegen ihrer leichten Beschaffbarkeit und günstigen Ergebnisse sind die Bromide des Lithiums, Natriums und Kaliums bevorzugt.

Die Reaktion der Verbindung der Formel (I) mit dem Bromid wird in einem dipolaren, aprotischen Losungsmittel durchgeführt. Hierbei werden auf 1 g der Verbindung der Formel (I) 0,1 bis 20 cm³ Lösungsmittel verwendet. Diese Grenzen sind nicht kritisch, es können beispielsweise je 1 g Verbindung der Formel (I) auch mehr als 20 cm³ Lösungsmittel eingesetzt werden, doch genügt es im allgemeinen, eine Lösungsmittelmenge, die in dem genannten Bereich liegt, zu wählen, vorzugsweise werden 0,5 bis 10 cm³ Losungsmittel je 1 g Verbindung der Formel (I) angewendet. Geeignete dipolare, aprotische Lösungsmittel sind zum Beispiel N-Methylpyrrolidon, Dimethylsulfoxid oder Tetramethylensulfon, vorzugsweise werden Dialkylcarbonsäureamide und insbesondere Dimethylformamid oder Dimethylacetamid eingesetzt. Es können auch Mischungen verschiedener dipolarer, aprotischer Lösungsmittel Anwendung finden.

Die oben beschriebene erfindungsgemäße Reaktion wird bei einer Temperatur von 120 bis 200 °C durchgeführt, wobei sie zweckmäßig unter normalem Atmosphärendruck oder unter dem autogenen Druck der Reaktionsmischung stattfindet. Die Anwendung höherer Drucke ist in der Regel nicht erforderlich und stellt einen unnötigen Aufwand dar. Unterhalb 120 °C verläuft die Reaktion im allgemeinen zu langsam, oberhalb 200 °C wird vermehrt die Bildung unerwünschter Nebenprodukte festgestellt. Soll die Reaktion im oberen Temperaturbereich, beispielsweise von 160 bis 200 °C, durchgeführt werden, wird zweckmäßig Dimethylacetamid anstelle Dimethylformamid verwendet. Vorzugsweise wird bei Temperaturen von 130 bis 180 °C gearbeitet.

Die Reaktionsdauer hängt von der angewendeten Temperatur und den eingesetzten Ausgangsstoffen ab und beträgt im allgemeinen 1 bis 20 Stunden; längere Reaktionsdauern sind möglich, jedoch wird meist kein zusätzlicher Effekt beobachtet, der die immer schlechter werdenden Raum-Zeit-Ausbeuten rechtfertigen würde. Häufig werden bei einer Reaktionsdauer von 2 bis 10 Stunden gute Ergebnisse erhalten.

Die erfindungsgemäße Reaktion wird vorteilhaft unter Ausschluß von Sauerstoff in einem Inertgas, beispielsweise Stickstoff oder Argon, durchgeführt.

Nach Beendigung der Reaktion kann die Mischung auf unterschiedliche Weise aufgearbeitet werden, beispielsweise durch Wasserdampfdestillation oder durch Abkühlen auf Raumtemperatur, Zugabe von Wasser und Abtrennen der Phase, die die organischen Stoffe enthält. In beiden Fällen kann die erhaltene Mischung organischer Stoffe zweckmäßigerweise nach Trocknung mit einem üblichen Trockenmittel einer fraktionierten Destillation bei Sumpftemperaturen bis maximal etwa 200 °C unterworfen werden, wobei, wenn erforderlich, verminderter Druck angewendet wird.

Es wurde ferner gefunden, daß die oben beschriebene Reaktion bei niedrigerer Temperatur und teilweise unter Erhalt besserer Ausbeuten in Gegenwart eines Alkalisalzes mindestens einer Hydroxyalkansulfinsäure mit 1 bis 5 C-Atomen durchgeführt werden kann. Vorzugsweise wird eine Hydroxyalkansulfinsäure mit 1 bis 3 C-Atomen verwendet, als Alkalisalze werden die Natrium- oder Kaliumsalze eingesetzt. Besonders gute Ergebnisse werden mit dem Natriumsalz der Hydroxymethansulfinsäure erhalten.

Bei der Umsetzung in Gegenwart von Hydroxyalkansulfinaten werden von diesen 0,2 bis 2 mol, vorzugsweise 0,5 bis 1,5 mol, je 1 mol gebundenes Iodatom in der Verbindung der Formel (I) angewendet. Die Reaktionstemperatur ist abhängig von den eingesetzten Ausgangsstoffen und beträgt -10 bis +120 °C. Unter -10 °C verlauft die Reaktion im allgemeinen zu langsam, oberhalb +120 °C nehmen unerwünschte Nebenreaktionen zu, vorzugsweise wird bei Temperaturen von 0 bis +40 °C gearbeitet. Für den Druck gilt das weiter oben Gesagte. Das Alkalisalz der Hydroxyalkansulfinsäure wird zweckmäßig als Pulver oder als Suspension in den obengenannten Lösungsmitteln eingesetzt, wobei die Lösungsmittelmenge zweckmäßig so bemessen wird, daß der Ansatz gut rührbar bleibt.

Es hat sich als vorteilhaft gezeigt, wenn nicht die Gesamtmenge des vorgesehenen Alkalisalzes der Hydroxyalkansulfinsäure zu Beginn der Reaktion zugesetzt wird, sondern nur ein Teil und die Restmenge kontinuierlich oder absatzweise (portionsweise) während des Verlaufs der Reaktion.

Die Reaktionsdauer hängt, wie oben bereits ausgeführt, von der gewählten Reaktionstemperatur und den Einsatzstoffen ab; sie ist infolge der niedrigeren Umsetzungstemperatur im allgemeinen höher als bei dem weiter oben beschriebenen Verfahren ohne Einsatz des Alkalisalzes der Hydroxyalkansulfinsäure. Wenn dieses eingesetzt wird, beträgt die Reaktionsdauer im allgemeinen 5 bis 100 Stunden, wobei häufig gute Ergebnisse im Bereich von 7 bis 50 Stunden erhalten werden. - Die Aufarbeitung zum Erhalt der weitgehend fluorierten Alkylbromide und eine eventuell notwendige Reinigung erfolgt wie weiter oben beschrieben.

Die nach dem erfindungsgemäßen Verfahren erzeugten, weitgehend fluorierten Alkylbromide können im medizinischen Sektor eingesetzt werden, wie zum Beispiel als Kontrastmittel bei Untersuchungen mit Röntgenstrahlen oder mit Ultraschall, beispielsweise zur Sichtbarmachung von Tumoren, zur Organperfusion sowie in wäßriger Emulsion als Blutersatzstoff. Weitere Anwendungen der weitgehend fluorierten Alkylbromide sind Hochtemperatur-Inertflüssigkeiten und Kontrastmittel für ¹⁹F-Kernresonanz-Spektral-(NMR)-Analyse.

Im Gegensatz zu den bekannten Verfahren ermöglicht es das erfindungsgemäße Verfahren in wenig aufwendigen Apparaturen ohne Schwierigkeiten bezüglich Korrosion und besondere Sicherheitsvorkehrungen zu arbeiten. Bei dem neuen Verfahren fallen Nebenprodukte an, die auf üblichem Wege isoliert werden können und verschiedene Verwendungen haben, beispielsweise die Verbindungen, die anstelle des Iodatoms in obengenannter Formel (I) ein Wasserstoffatom tragen, können als Wärmeträgerflüssigkeiten verwendet werden, und Verbindungen, die anstelle der -CF₂I-Gruppe in obengenannter Formel (I) eine -COOH-Gruppe tragen beziehungsweise deren Salze, werden beispielsweise als chemisch beständige Emulgatoren eingesetzt.

Nachfolgende Beispiele sollen die Erfindung näher erläutern.

### Beispiel 1

In einem Rundkolben mit 500 cm³ Inhalt, der mit einem Innenthermometer und einem Rührer aus Glas ausgerüstet ist, werden 54,6 g (0,1 mol) 1-Iod-perfluoroctan und 15,4 g (0,15 mol) wasserfreies Natriumbromid eingewogen und nach Zugabe von 50 cm³ Dimethylacetamid in einer Stickstoffatmosphäre unter Rühren auf 156 °C erwärmt. Auf 1 mol gebundenes Iodatom im 1-Iod-perfluoroctan [= Verbindung der Formel (I)] werden 0,15 mol Bromidionen und je 1 g Verbindung der Formel (I) 0,92 cm³ dipolares, aprotisches Lösungsmittel eingesetzt. Während 6 Stunden Reaktionszeit sinkt die Temperatur auf 142 °C, anschließend wird die Reaktionsmischung auf Raumtemperatur abgekühlt, der Kolbeninhalt mit Wasser versetzt und in einen Scheidetrichter gegeben. In diesem scheidet sich eine flüssige fluororganische Unterphase ab. Diese wird mit einer wäßrigen Lösung, die 20 Gew.-% NaOH enthält, anschließend mit Wasser ausgeschüttelt und getrocknet. Es werden 47,4 g eines Rohproduktes erhalten, das ¹⁹F-NMR-spektroskopisch untersucht wird. Die Ausbeuten, bezogen auf eingesetztes 1-Iod-perfluoroctan, betragen: 29 % 1-Brom-perfluoroctan; 17 % 1-Hydro-perfluoroctan und 48 % des Ausgangsproduktes 1-Iod-perfluoroctan.

### Beispiel 2

Es wird verfahren wie in Beispiel 1 beschrieben, jedoch werden anstelle des Natriumbromids 13 g (0,15 mol) Lithiumbromid und anstelle des Dimethylacetamids Dimethylformamid eingesetzt. Die Molverhältnisse der Verbindung der Formel (I) zu Bromid beziehungsweise zu cm³ Lösungsmittel bleiben die gleichen. Die Reaktionsmischung wird auf 158 °C erwärmt, die Temperatur sinkt während 6 Stunden auf 133 °C, dann wird abgekühlt und aufgearbeitet, wie in Beispiel 1 beschrieben. Es werden 47,5 g Rohprodukt erhalten, das ¹⁹F-NMR-spektroskopisch untersucht wird. Die Ausbeuten, bezogen auf eingesetztes 1-Iod-perfluoroctan, betragen: 38 % 1-Brom-perfluoroctan; 9 % 1-Hydro-perfluoroctan und 45 % des Ausgangsproduktes 1-Iod-perfluoroctan.

### Beispiel 3

Es wird verfahren wie in Beispiel 1 beschrieben, jedoch werden anstelle des Natriumbromids 21,5 g (0,15 mol) Kupfer-(I)-bromid und anstelle 50 cm³ Dimethylacetamid 100 cm³ Dimethylformamid eingesetzt. Auf 1 mol gebundenes Iodatom in der Verbindung der Formel (I) werden 1,5 mol Bromidionen und auf 1 g der Verbindung der Formel (I) 1,83 cm³ polares, aprotisches Lösungsmittel verwendet. Die Mischung wird unter Rühren auf 138 °C erwärmt und während 14 Stunden sinkt die Temperatur auf 129 °C. Dann wird abgekühlt, unter Zwischenschaltung einer Kältefalle mit Unterdruck abfiltriert und das Filtrat aufgearbeitet, wie in Beispiel 1 beschrieben. Es werden 38,0 g Rohprodukt erhalten, das ¹⁹F-NMR-spektroskopisch untersucht wird. Die Ausbeuten, bezogen auf eingesetztes 1-Iod-perfluoroctan, betragen: 32 % 1-Brom-perfluoroctan; 7,6 % 1-Hydro-perfluoroctan und 34 % des Ausgangsproduktes 1-Iod-perfluoroctan.

### Beispiel 4

Es wird verfahren wie in Beispiel 1 beschrieben, jedoch werden anstelle 50 cm³ Dimethylacetamid 100 cm³ Dimethylformamid und zusätzlich 15,4 g Dihydrat des Natriumsalzes der Hydroxymethansulfinsäure (ein käufliches Produkt) eingesetzt. Auf 1 mol gebundenes Iodatom in der Verbindung der Formel (I) werden 1,5 mol Bromidionen und 1 mol Dihydrat des Natriumsalzes der Hydroxymethansulfinsäure sowie je 1 g der Verbindung der Formel (I) 1,83 cm³ polares, aprotisches Lösungsmittel verwendet. Die Reaktionsmischung wird unter Rühren auf +10 °C abgekühlt und während 14 Stunden auf dieser Temperatur gehalten. Nach Ablauf der Reaktionszeit wird auf Raumtemperatur erwärmt und aufgearbeitet, wie in Beispiel 1 beschrieben, wobei sich die fluororganische Phase im Scheidetrichter nur langsam nach mehreren Stunden stehenlassen abscheidet. Es werden so 34,5 g Rohprodukt erhalten, das nach ¹⁹F-NMR-Analyse untersucht wird. Die Ausbeuten, bezogen auf eingesetztes 1-Iod-perfluoroctan, betragen: 48 % 1-Brom-perfluoroctan; 1 % 1-Hydro-perfluoroctan und 18,5 % des Ausgangsproduktes 1-Iod-perfluoroctan.

### Beispiel 5

Es wird verfahren wie in Beispiel 4 beschrieben, jedoch werden anstelle 15,4 g Natriumbromid diesmal 41,2 g (0,4 mol) Natriumbromid und anstelle 100 cm³ Dimethylformamid diesmal 300 cm³ Dimethylformamid eingesetzt. Die Zugabemenge des Dihydrates vom Natriumsalz der Hydroxymethansulfinsäure bleibt die gleiche. Je 1 mol gebundenes Iodatom in der Verbindung der Formel (I) werden 4 mol Bromidionen, 1 mol Dihydrat des Natriumsalzes der Hydroxyalkansulfinsäure und je 1 g Verbindung der Formel (I) 5,49 cm³ polares, aprotisches Losungsmittel verwendet. Die Reaktionsmischung wird während 6 Stunden bei Raumtemperatur (25 °C) gerührt und anschließend aufgearbeitet, wie in Beispiel 1 beschrieben, wobei sich wiederum die fluororganische Phase im Scheidetrichter nur langsam nach mehreren Stunden absetzt. Es werden 39,4 g eines Rohproduktes erhalten, das ¹⁹F-NMR-spektroskopisch untersucht wird. Die Ausbeuten, bezogen auf eingesetztes 1-Iod-perfluoroctan, betragen: 40 % 1-Brom-perfluoroctan; 3 % 1-Hydro-perfluoroctan und 33 % des Ausgangsproduktes 1-Iod-perfluoroctan.

### Beispiel 6

In einem Rundkolben von 1 dm³ Inhalt, der mit einem Innenthermometer und einem Glasrührer ausgerüstet ist, werden 54,6 g (0,1 mol) 1-Iod-perfluoroctan und 20,6 g (0,2 mol) wasserfreies Natriumbromid gegeben und nach Zusatz von 500 cm³ Dimethylformamid unter einer Stickstoffatmosphäre gerührt, wobei die Temperatur des Kolbeninhaltes mit einem Kryostaten während 48 Stunden bei 0 °C gehalten wird. Zu Beginn der Reaktion - nach 8 Stunden, nach 24 Stunden und nach 32 Stunden - werden in vier gleichen Portionen je 7,7 g (0,05 mol) Dihydrat des Natriumsalzes der Hydroxymethansulfinsäure zugegeben. Nach 48 Stunden wird die Reaktionsmischung auf Raumtemperatur erwärmt, mit Wasser versetzt und aufgearbeitet, wie in Beispiel 1 beschrieben. Wiederum wird ein nur langsames Absetzen der fluororganischen Phase im Scheidetrichter beobachtet. Eine mögliche Ursache hierfür ist die bei der Umsetzung mit entstandene und als Emulgator wirkende Perfluoroctansäure. Es werden 31,0 g eines Rohproduktes erhalten, das nach ¹⁹F-NMR-spektroskopischer und gaschromatographischer Analyse 94 Gew.-% 1-Brom-perfluoroctan; 2 Gew.-% 1-Hydro-perfluoroctan und 1,5 Gew.-% des Ausgangsproduktes 1-Iod-perfluoroctan enthält. - Je 1 mol gebundenes Iodatom in der Verbindung der Formel (I) werden 2 mol Bromidionen und 2 mol Dihydrat des Natriumsalzes der Hydroxymethansulfinsäure sowie je 1 g Verbindung der Formel (I) 9,16 cm³ polares, aprotisches Lösungsmittel eingesetzt.

### Beispiel 7

In einem Rundkolben von 2 dm³ Inhalt, der mit Innenthermometer und Glasrührer ausgerüstet ist, werden 273 g (0,5 mol) 1-Iod-perfluoroctan, 102,9 g (1 mol) wasserfreies Natriumbromid und 1 000 cm³ Dimethylformamid eingebracht. Der Kolbeninhalt wird unter Rühren mit Stickstoff gespült und während der gesamten Reaktionszeit von 48 Stunden auf einer Temperatur von 15 °C gehalten. Zu Beginn der Reaktion - nach 8 Stunden und nach 24 Stunden - werden jeweils 38,5 g Portionen (je 0,25 mol) Dihydrat des Natriumsalzes der Hydroxymethansulfinsäure zugesetzt. Nach Ablauf der Reaktionszeit wird der Kolbeninhalt auf Raumtemperatur erwärmt, mit 1 dm³ Wasser versetzt und mit einer wäßrigen Lösung, die 30 Gew.-% NaOH enthält, auf pH 11 eingestellt. Der Kolbeninhalt wird nun einer Wasserdampfdestillation (Sumpftemperatur 99 bis 108 °C, Übergangstemperatur 92 bis 102 °C) unterworfen, bis sich in der Vorlage keine fluororganische Unterphase mehr abscheidet. Das aus dem Sumpf abdestillierte Wasser wird über einen Tropftrichter wieder ersetzt. Die fluororganische Unterphase in der Destillationsvorlage wird in einem Scheidetrichter von der oberen wäßrigen Phase abgetrennt, dann bei 95 °C mit einer wäßrigen Losung, die 20 Gew.-% NaOH enthält, verrührt, anschließend mit Wasser ausgeschüttelt und getrocknet. Es werden 166 g eines Rohproduktes erhalten, das nach gaschromatographischer Analyse 87,1 % 1-Brom-perfluoroctan und 10,9 % 1-Hydro-perfluoroctan enthält. Dies entspricht einer Rohproduktausbeute von 58 % 1-Brom-perfluoroctan. Von diesem Rohprodukt werden 160 g unter normalem Atmosphärendruck in einer Spaltrohrkolonne destilliert. Als Hauptlauf werden 93 g 1-Brom-perfluoroctan mit einem Siedepunkt von 140 bis 141 °C erhalten. Dies entspricht, unter Berücksichtigung, daß nicht alles Rohprodukt zur Destillation eingesetzt wurde, einer Ausbeute von 39 %, bezogen auf eingesetztes 1-Iod-perfluoroctan. Durch eine zweite Destillation der Destillationsvorläufe und -ruckstände kann noch weiteres 1-Brom-perfluoroctan gewonnen werden. - Je 1 mol gebundenes Iodatom in der Verbindung der Formel (I) werden 2 mol Bromidionen und 1,5 mol Dihydrat des Natriumsalzes der Hydroxymethansulfinsäure sowie je 1 g Verbindung der Formel (I) 3,66 cm³ polares, aprotisches Lösungsmittel eingesetzt.

## Patentansprüche

1. Verfahren zur Herstellung von weitgehend fluorierten Alkylbromiden aus Verbindungen der Formel
X-(CF₂)ₙ-I (I),
in der bedeuten: X = H, F, Cl, Br, I oder (CF₃)₂CF- und n = 1 bis 16, dadurch gekennzeichnet, daß in mindestens einem dipolaren, aprotischen Lösungsmittel 1 mol gebundenes Iodatom der Verbindung der Formel (I) mit 1 bis 4 mol Bromidionen, die als Salze mit mindestens einem der folgenden Kationen: Alkalimetall, Erdalkalimetall, Kupfer, substituiertes oder unsubstituiertes Ammonium vorliegen, bei 120 bis 200 °C unter normalem Atmosphärendruck oder unter dem autogenen Druck der Reaktionsmischung umgesetzt werden.

2. Verfahren nach Anspruch 1, dadurch gekennzeichnet, daß die Umsetzung in Gegenwart von 0,2 bis 2 mol von einem Alkalisalz mindestens einer Hydroxyalkansulfinsäure mit 1 bis 5 C-Atomen je 1 mol gebundenes Iodatom der Verbindung der Formel (I) bei einer Temperatur von -10 bis +120 °C durchgeführt wird.

3. Verfahren nach Anspruch 2, dadurch gekennzeichnet, daß das Alkalisalz der Hydroxyalkansulfinsäure während der Umsetzung der Reaktionsmischung kontinuierlich oder absatzweise zugegeben wird.

4. Verfahren nach einem oder mehreren der Ansprüche 1 bis 3, dadurch gekennzeichnet, daß mindestens ein Perfluoralkyliodid mit 4 bis 12 C-Atomen eingesetzt wird.

5. Verfahren nach einem oder mehreren der Ansprüche 1 bis 4, dadurch gekennzeichnet, daß Lithium-, Natrium- oder Kaliumbromid eingesetzt wird.

6. Verfahren nach einem oder mehreren der Ansprüche 1 bis 5, dadurch gekennzeichnet, daß als Lösungsmittel Dimethylformamid oder Dimethylacetamid oder deren Gemische eingesetzt werden.

7. Verfahren nach einem oder mehreren der Ansprüche 1 und 4 bis 6, dadurch gekennzeichnet, daß die Umsetzung bei einer Temperatur von 130 bis 180 °C erfolgt.

8. Verfahren nach einem oder mehreren der Ansprüche 2 bis 6, dadurch gekennzeichnet, daß die Umsetzung bei einer Temperatur von 0 bis 40 °C erfolgt.

9. Verfahren nach einem oder mehreren der Ansprüche 1 bis 8, dadurch gekennzeichnet, daß je 1 g Verbindung der Formel (I) 0,1 bis 20 ccm Lösungsmittel verwendet werden.

## Claims

1. A process for the preparation of an extensively fluorinated alkyl bromide from a compound of the formula
X-(CF₂)ₙ-I (I)
in which: X is H, F, Cl, Br, I or (CF₃)₂CF- and n is 1 to 16 comprises reacting, in at least one dipolar aprotic solvent, 1 mol of bonded iodine atom of the compound of the formula (I) with 1 to 4 mol of bromide ions which are present as salts with at least one of the following cations: alkali metal, alkaline earth metal, copper or substituted or unsubstituted ammonium, at 120 to 200°C under normal atmospheric pressure or under the autogenous pressure of the reaction mixture.

2. The process as claimed in claim 1, wherein the reaction is carried out in the presence of 0.2 to 2 mol of an alkali metal salt of at least one hydroxyalkane-sulfinic acid having 1 to 5 carbon atoms per mol of bonded iodine atom of the compound of the formula (I) at a temperature of -10 to +120°C.

3. The process as claimed in claim 2, wherein the alkali metal salt of the hydroxyalkanesulfinic acid is added to the reaction mixture continuously or batchwise during the reaction.

4. The process as claimed in one or more of claims 1 to 3, wherein at least one perfluoroalkyl iodide having 4 to 12 carbon atoms is employed.

5. The process as claimed in one or more of claims 1 to 4, wherein lithium bromide, sodium bromide or potassium bromide is employed.

6. The process as claimed in one or more of claims 1 to 5, wherein dimethylformamide or dimethylacetamide or a mixture thereof is employed as the solvent.

7. The process as claimed in one or more of claims 1 and 4 to 6, wherein the reaction is carried out at a temperature of 130 to 180°C.

8. The process as claimed in one or more of claims 2 to 6, wherein the reaction is carried out at a temperature of 0 to 40°C.

9. The process as claimed in one or more of claims 1 to 8, wherein 0.1 to 20 ccm of solvent are used per g of compound of the formula (I).

## Revendications

1. Procédé pour préparer des bromures d'alkyles fortement fluorés à partir de composés de formule :
X-(CF₂)ₙ-I (I),
(dans laquelle X représente H, F, Cl, Br, I ou un groupe (CF₃)₂C_{F}- et n vaut 1 à 16), procédé caractérisé en ce qu'on fait réagir à 120 jusqu'à 200°C, sous la pression atmosphérique normale ou sous la pression autogène du mélange réactionnel, dans au moins un solvant aprotique dipolaire, une mole d'un atome d'iode lié du composé de formule (I) avec 1 à 4 moles d'ions bromures, qui est ou sont présent(s)sous forme de sels avec au moins l'un des cations suivants : métal alcalin, métal alcalino terreux, cuivre, groupe ammonium substitué ou non substitué.

2. Procédé selon la revendication 1, caractérisé en ce qu'on conduit la réaction en présence de 0,2 à 2 moles d'un sel alcalin d'au moins un acide hydroxyalcanesulfinique comportant 1 à 5 atomes de carbone par mole d'atome d'iode lié du composé de formule (I), en opérant à une température de -10 jusqu'à + 120°C.

3. Procédé selon la revendication 2, caractérisé en qu'on ajoute, en continu ou par fractions, le sel alcalin de l'acide hydroxyalcanesulfinique pendant la réaction du mélange réactionnel.

4. Procédé selon l'une ou plusieurs des revendications 1 à 3, caractérisé en ce qu'on utilise au moins un iodure de perfluoralkyle comportant 4 à 12 atomes de carbone.

5. Procédé selon une ou plusieurs des revendications 1 à 4, caractérisé en ce qu'on utilise du bromure de lithium, de sodium ou de potassium.

6. Procédé selon l'une ou plusieurs des revendications 1 à 5, caractérisé en ce qu'on utilise comme solvant le diméthylformamide ou le diméthylacétamide ou leurs mélanges.

7. Procédé selon une ou plusieurs des revendications 1 et 4 à 6, caractérisé en ce que la réaction a lieu à une température de 130 à 180°C.

8. Procédé selon une ou plusieurs des revendications 2 à 6, caractérisé en ce que la réaction a lieu à une température de 0 à 40°C.

9. Procédé selon une ou plusieurs des revendications 1 à 8, caractérisé en ce qu'on utilise, pour chaque gramme du composé de formule (I), 0,1 à 20 cm³ de solvant.
